# EUROPÄISCHE PATENTANMELDUNG

(11) **EP 1 800 668 A1**
(43) Veröffentlichungstag der Anmeldung: **27.06.2007**
(21) Anmeldenummer: 06001288.7
(22) Anmeldetag: 23.01.2006
(51) Int. Cl.: A61K 9/16, A61K 31/70

(54) **Verfahren zur Herstellung von Antibiotikum-/Antibiotika-Partikeln und deren Verwendung**

(30) Priorität: 20.12.2005 DE 102005061290
(71) Anmelder: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(57) **Zusammenfassung**

Verfahren zur Herstellung von Antibiotikum-/Antibiotika-Partikeln dadurch gekennzeichnet, dass eine wässrige Lösung eines Aminoglykosid-Antibiotikums oder eine wässrige Lösung von zwei oder mehr Aminoglykosid-Antibiotika unter Rühren mit einem Lösungsmittelgemisch aus Isopropanol und mindestens einem weiteren Alkohol vermischt wird, wobei das Volumenverhältnis Lösungsmittelgemisch zu wässriger Lösung mindestens 3 zu 1 ist und wobei die entstandene Suspension so lange gerührt wird, bis die primär entstandenen groben Antibiotikum-/Antibiotika-Aggregate in Partikel zerfallen sind, die eine Korngröße kleiner 400 µm besitzen.

## Beschreibung

Gegenstand der Erfindung ist ein Verfahren zur Herstellung von Antibiotikum-/Antibiotika-Partikeln und deren Verwendung. Unter Antibiotikum-/Antibiotika-Partikeln werden hierbei Partikel verstanden, die eine Korngröße bis etwa 400 µm, insbesondere im Bereich von 63-400 µm, haben.

Aminoglykosid-Antibiotika sind typische, bakterizide Breitbandantibiotika mit einem ausgeprägten postantibiotischen Effekt. Aminoglykosid-Antibiotika, insbesondere Gentamicin in Form von Gentamicinsulfat, werden zum antibiotischen Schutz von Polymethylmethacylat-Knochen-zementen eingesetzt (K.-D. Kühn: Bone Cements, Springer-Verlag, 2000). Polymethylmeth-acrylat-Knochenzemente dienen in der Chirurgie und Orthopädie zur Fixierung von Endo-prothesen. Dabei besteht die Gefahr, dass nach Aushärtung des Knochenzementes an der Grenzfläche zwischen dem Knochengewebe und dem Polymethylmethacrylat-Knochenzement eine Keimbesiedelung eintreten kann. Ein wirksamer Schutz gegen eine bakterielle Keimbesiedelung kann durch in den Knochenzement eingebrachte bakterizide Antibiotika erreicht werden, die durch Einwirkung des wässrigen physiologischen Milieus aus dem ausgehärteten Polymethylmethacrylat-Knochenzement, herausgelöst werden und an der Grenzfläche zwischen dem Knochengewebe und dem Polymethylmethacrylat-Knochenzement eine Abtötung empfindlicher bakterieller Keime bewirken.
Aminoglykosid-Antibiotika werden biotechnologisch beziehungsweise partial-synthetisch unter Verwendung von Micromonospora Arten, wie zum Beispiel *Micromonospora purpurea,* industriell hergestellt. Dabei fallen die Aminoglykosid-Antibiotika auf Grund des zumeist angewandten Sprühtrocknungsverfahrens als sehr feine Pulver mit typischen Korngrößen kleiner 64 µm an. Es hat sich jedoch gezeigt, dass für eine optimale Freisetzung von Antibiotika aus Polymethylmethacyrlat-Knochenzementen gröbere Partikel vorteilhafter sind. Optimal ist eine Korngröße im Bereich von 63-250 µm.

Aminoglykosid-Antibiotika werden im allgemeinen in Form der Sulfate verwendet und lösen sich problemlos in Wasser. Aus den US 3,136,704 und US 3,091,572 ist bekannt, das Gentamicinsulfat aus der wässrigen Lösung mit Methanol gefällt werden kann. Es zeigte sich jedoch in eigenen Versuchen, dass kein körniger, leicht zu isolierender, grober Niederschlag durch Vermischung von Methanol mit wässrigen Gentamicinsulfat-Lösungen entsteht. Beim Vermischen von wässrigen Gentamcinsulfat-Lösungen mit Methanol oder anderen niederen Alkoholen scheidet sich das Gentamicinsulfat in Form einer extrem klebrigen, schleimartigen Schicht am Boden des Reaktionsgefäßes ab. Es bildet sich an der Grenzfläche zwischen der Schicht und der darüber befindlichen Lösung eine Haut, die eine Entwässerung des Gentamicinsulfat-Schleims behindert oder unmöglich macht. Erst nach mehreren Tagen bis Wochen erstarrt die Schicht. Diese Schicht aus Gentamicinsulfat kann weiter getrocknet werden und anschließend nach Brechen auf die gewünschte Korngröße fraktioniert werden. Die Herstellung von partikulärem Gentamicinsulfat ist jedoch nach dieser Verfahrensweise wirtschaftlich nicht sinnvoll.
Ein weitgehend gleiches Verhalten zeigen auch die Sulfate und Chloride der Antibiotika Tobramycin, Amikacin, Netilmicin, Sisomycin und Kanamycin.

Die Aufgabe der Erfindung besteht darin, ein Verfahren zu entwickeln, mit dem es möglich ist, in wirtschaftlich vorteilhafter, einfacher Weise Aminoglykosid-Antibiotika in eine partikuläre Form mit einer Korngröße bis 400 µm, insbesondere im Bereich von 63-400 µm, zu überführen.

Die Aufgabe wurde dadurch gelöst, dass eine wässrige Lösung eines Aminoglykosid-Antibiotikums oder eine wässrige Lösung von zwei oder mehr Aminoglykosid-Antibiotika unter Rühren mit einem Lösungsmittelgemisch aus Isopropanol und mindestens einem weiteren Alkohol vermischt wird, wobei das Volumenverhältnis Lösungsmittelgemisch zu wässriger Lösung mindestens 3 zu 1 ist und wobei die entstandene Suspension so lange gerührt wird oder nicht gerührt belassen wird, bis die primär entstandenen groben Antibiotikum-/Antibiotika-Aggregate in Partikel zerfallen sind, die eine Korngröße kleiner 400 µm besitzen.

Die Erfindung beruht auf dem überraschenden Befund, dass Gemische aus 30-70 Volumenprozent Isopropanol und 30-70 Volumenprozent Methanol beim Vermischen mit wässrigen Lösungen von Aminoglykosid-Antibiotika zuerst grobe Antibiotika-Aggregate, mit einer Größe von ungefähr 1-10 mm, bilden, die ohne äußere Einwirkung oder auch beschleunigt durch Rühren in Partikel mit einer Korngröße < 400 µm zerfallen, wobei hauptsächlich Partikel mit einer Korngröße von 100-250 µm entstehen. Der Zerfallsprozess läuft sowohl bei Raumtemperatur als auch bei erhöhter Temperatur ab. Als optimal hat sich ein Gemisch von 50 Volumenprozent Isopropanol und 50 Volumenprozent Methanol erwiesen. Unter Verwendung dieses Gemischs zerfallen die primär gebildeten Antibiotika-Aggregate innerhalb von wenigen Minuten bis zu wenigen Stunden. Die gebildeten Antibiotikum-/Antibiotika-Partikel lassen sich abfiltrieren oder zentrifugieren und können nach weiteren Trocknungsschritten durch Siebung fraktioniert werden. Dadurch wird z.B. die bevorzugte Siebfraktion mit Korngrößen von 63-400 µm erhalten.

Es ist im Rahmen des Verfahrens auch möglich, den Zerfall der primär gebildeten Antibiotika-Aggregate bei erhöhter Temperatur ablaufen zu lassen.

Als der - neben Isopropanol - weitere Alkohol ist Methanol bevorzugt.

Demgemäß wird ein Lösungsmittelgemisch aus 30-70 Volumenprozent Isopropanol und 70-30 Volumenprozent Methanol bevorzugt.

Besonders bevorzugt ist ein Lösungsmittelgemisch aus 50 Volumenprozent Isopropanol und 50 Volumenprozent Methanol.

Das Aminoglykosid-Antibiotikum oder die Aminoglykosid-Antibiotika entstammen vorzugsweise der Gruppe bestehend aus Gentamicin, Tobramycin, Amikacin, Kanamycin, Netilmicin und Sisomycin.

Die wässrige Lösung weist bevorzugt einen Antibiotikum-/Antibiotika-Gehalt von 30-60 Masseprozent auf. Lösungen mit niedrigeren Antibiotikum-/Antibiotika-Gehalten sind unwirtschaftlich und ergeben mitunter zu feine Partikel. Bei höheren Antibiotikum-/Antibiotika-Gehalten sind die wässrigen Lösungen hoch viskos und schlecht zu handhaben. Insbesondere dauert der Zerfallsprozess der Primäraggregate deutlich länger als in dem bevorzugten Konzentrationsbereich. Als besonders vorteilhaft hat sich die Verwendung von wässrigen Lösungen mit einem Antibiotikum-/Antibiotika-Gehalt von 50 Masseprozent erwiesen.

Die wässrige Lösung kann neben einem oder mehreren Aminoglykosid-Antibiotika gegebenenfalls ein oder mehrere Antibiotika aus den Gruppen der Glykopeptid-Antibiotika, der 4-Chinolonantibiotika, der Lincosamid-Antibiotika, der Makrolide, der Ketolide, der Glycylcycline und des Linezolids oder davon abgeleiteter antimikrobiell wirksamer Derivate enthalten. Dadurch lassen sich vorteilhaft Antibiotika-Partikel fertigen, die sich dadurch auszeichnen, dass bei ihrer Verwendung in Polymethylmethacrylat-Knochenzementen eine synchrone Freisetzung von zwei oder mehreren Antibiotika nach radikalischer Aushärtung der Knochenzemente möglich ist. Dadurch können optimale Wirkstoffspiegel insbesondere von synergistisch wirkenden Antibiotika synchron erreicht werden.

Die wässrige Lösung kann auch zusätzlich ein oder mehrere Polymere aus den Gruppen des Polyvinylpyrolidons, des Vinylalkohols, der Gelatine, der Carboxymethylcellulose, der Hydroxy-ethylcellulose, der Methylcellulose und der Polyacrylsäuresalze in gelöster Form enthalten. Durch die zugesetzten Polymere kann das Rieselverhalten der gebildeten Antibiotika-Partikel beeinflusst werden.

Im Rahmen des erfindungsgemäßen Verfahrens ist es auch, wenn in der wässrigen Antibiotikum-/Antibiotika-Lösung gegebenenfalls noch ungelöste Antibiotikum-/Antibiotika-Primärpartikel suspendiert sind. Bei der technischen Herstellung von Antibiotika-Partikeln ist es durchaus auch möglich, dass nicht sichtklare Antibiotikum- oder Antibiotika-Lösungen eingesetzt werden. Es zeigte sich überraschend, dass noch vorhandene Reste von Primärpartikeln die Bildung der gewünschten groben Antibiotika-Partikel nicht negativ beeinflussen.

Die Erfindung betrifft auch die Verwendung von nach dem erfindungsgemäßen Verfahren hergestellten Antibiotika-Partikeln zur antibiotischen Ausrüstung von Medizinprodukten und Arzneimitteln, die zur lokalen Freisetzung von einem Antibiotikum oder mehreren Antibiotika bestimmt sind. Besonders bevorzugt ist die Verwendung der erfindungsgemäßen Antibiotika-Partikel zur antibiotischen Ausrüstung von Polymethylmethacrylat-Knochenzementen.

Die Erfindung wird durch die nachstehenden Beispiele näher erläutert, ohne jedoch die Erfindung zu beschränken. Teile und Prozentangaben beziehen sich auf das Gewicht, sofern nicht anders angegeben.

Abbildung:

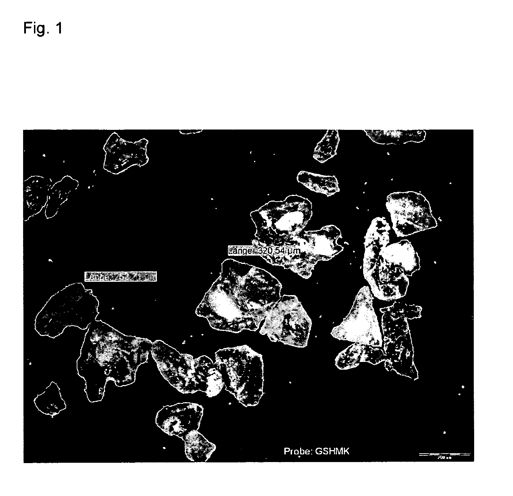

Fig. 1 zeigt eine lichtmikroskopische Aufnahme des Produkts des folgenden Beispiels 1.

### Beispiel 1

Es werden 10,0 g Gentamicinsulfat (AK 648, Fujiang/Fukang) in 10,0 g destilliertem, pyrogen-freiem Wasser gelöst. Diese Lösung wird unter Rühren in ein Gemisch aus 50 ml Isopropanol und 50 ml Methanol getropft. Es bilden sich innerhalb weniger Sekunden Primäraggregate. Nach einer halben Stunde wird nicht mehr gerührt. Nach vier Stunden sind die sedimentierten Primäraggregate zerfallen. Die gebildeten Partikel werden abgesaugt, danach eine halbe Stunde in Methanol bei Raumtemperatur ausgerührt und dann bis zur Massekonstanz im Vakuum getrocknet. Die Gesamtausbeute beträgt 9,8 g. Nach Siebung werden 7,3 g Partikel mit einer Siebfraktion von 63-250 µm erhalten. In Fig. 1 ist eine lichtmikroskopische Aufnahme dieser Partikel wiedergegeben.

### Beispiel 2

Es werden 10,0 g Gentamicinsulfat (AK 648, Fujiang/Fukang) in 10,0 g destilliertem, pyrogen-freiem Wasser gelöst. Diese Lösung wird unter Rühren in ein unter Rückfluss kochendes Gemisch aus 50 ml Isopropanol und 50 ml Methanol getropft. Es bilden sich innerhalb weniger Sekunden Primäraggregate. Nach einer halben Stunde wird nicht mehr gerührt und das Lösungsmittelgemisch unter Rückfluss belassen. Die gebildeten Partikel werden nach 5 Stunden auf Raumtemperatur abgekühlt, abgesaugt, danach eine halbe Stunde in Methanol bei Raumtemperatur ausgerührt und dann bis zur Massekonstanz im Vakuum getrocknet. Die Gesamtausbeute beträgt 9,6 g. Nach Siebung werden 6,6 g Partikel der Siebfraktion von 63-250 µm erhalten.

### Beispiel 3

Es werden 7,7 g Gentamicinsulfat (AK 648, Fujiang/Fukang) und 5,8 g Clindamycinhydrochlorid (AK 862) in 10,0 g destilliertem, pyrogenfreiem Wasser gelöst. Diese Lösung wird unter Rühren in ein Gemisch aus 50 ml Isopropanol und 50 ml Methanol getropft. Es bilden sich innerhalb weniger Sekunden Primäraggregate. Nach einer halben Stunde wird nicht mehr gerührt. Nach fünf Stunden sind die sedimentierten Primäraggregate zerfallen. Die gebildeten Patikel werden abgesaugt, danach eine halbe Stunde in Methanol bei Raumtemperatur ausgerührt und dann bis zur Massekonstanz im Vakuum getrocknet. Die Gesamtausbeute beträgt 11,2 g. Nach Siebung werd 6,4 g Partikel der Siebfraktion 63-250 µm erhalten.

### Beispiel 4

Es werden 1,540 kg Gentamicinsulfat (AK 648, Fujiang/Fukang) in 1,0 kg destilliertem, pyrogen-freiem Wasser gelöst. In einem 30 Liter-Hängegefäß werden 5,0 kg Isopropanol und 5,0 kg Methanol vorgelegt. In diese Lösung wird die wässrige Lösung innerhalb von dreißig Minuten unter Rühren (50 Umdrehungen pro Minute) getropft. Es bilden sich beim Zutropfen innerhalb weniger Sekunden Primäraggregate. Nach vier Stunden Rühren bei Raumtemperatur sind die Primäraggregate zerfallen. Die gebildeten Partikel werden abgesaugt, danach eine Stunde in trockenem Methanol bei Raumtemperatur ausgerührt und dann bis zur Massekonstanz im Vakuum getrocknet. Die Gesamtausbeute beträgt 1,351 kg. Nach Siebung werden 0,899 kg Partikel der Siebfraktion 63-250 µm erhalten.

## Patentansprüche

1. Verfahren zur Herstellung von Antibiotikum-/Antibiotika-Partikeln, **dadurch gekennzeichnet, dass**
• eine wässrige Lösung eines Aminoglykosid-Antibiotikums oder eine wässrige Lösung von zwei oder mehr Aminoglykosid-Antibiotika unter Rühren mit einem Lösungsmittelgemisch aus Isopropanol und mindestens einem weiteren Alkohol vermischt wird, wobei das Volumenverhältnis Lösungsmittelgemisch zu wässriger Lösung mindestens 3 zu 1 ist
• und die entstandene Suspension so lange gerührt wird oder nicht gerührt belassen wird, bis die primär entstandenen groben Antibiotikum-/Antibiotika-Aggregate in Partikel zerfallen sind, die eine Korngröße kleiner 400 µm besitzen.

2. Verfahren nach Anspruch 1, **dadurch gekennzeichnet, dass** als Alkohol Methanol bevorzugt wird.

3. Verfahren nach einem der Ansprüche 1 und 2, **dadurch gekennzeichnet, dass** das Aminoglykosid-Antibiotikum oder die Aminoglykosid-Antibiotika aus der Gruppe bestehend aus Gentamicin, Tobramycin, Amikacin, Kanamycin, Netilmicin und Sisomycin ausgewählt sind.

4. Verfahren nach mindestens einem der vorstehenden Ansprüche, **dadurch gekennzeichnet, dass** ein Lösungsmittelgemisch aus 30-70 Volumenprozent Isopropanol und 70-30 Volumenprozent Methanol eingesetzt wird.

5. Verfahren nach mindestens einem der vorstehenden Ansprüche, **dadurch gekennzeichnet, dass** ein Lösungsmittelgemisch aus 50 Volumenprozent Isopropanol und 50 Volumenprozent Methanol eingesetzt wird.

6. Verfahren nach mindestens einem der Ansprüche 1 bis 5, **dadurch gekennzeichnet, dass** die wässrige Lösung einen Antibiotikum-/Antibiotika-Gehalt von 30-60 Masseprozent hat.

7. Verfahren nach mindestens einem der Ansprüche 1 bis 6, **dadurch gekennzeichnet, dass** die wässrige Lösung neben einem oder mehreren Aminoglykosid-Antibiotika zusätzlich ein oder mehrere Antibiotika aus den Gruppen der Glykopeptid-Antibiotika, der 4-Chinolonantibiotika, der Lincosamid-Antibiotika, der Makrolide, der Ketolide, der Glycylcycline und des Linezolids oder davon abgeleiteter antimikrobiell wirksamer Derivate enthält.

8. Verfahren nach mindestens einem der Ansprüche 1 bis 7, **dadurch gekennzeichnet, dass** die wässrige Lösung zusätzlich ein oder mehrere Polymere aus den Gruppen des Polyvinylpyrolidons, des Vinylalkohols, der Gelatine, der Carboxymethylcellulose, der Hydroxyethylcellulose, der Methylcellulose und der Polyacrylsäuresalze in gelöster Form enthält.

9. Verfahren nach mindestens einem der Ansprüche 1-8, **dadurch gekennzeichnet, dass** in der wässrigen Antibiotikum-/Antibiotika-Lösung noch ungelöste Antibiotikum-/Antibiotika-Primärpartikel suspendiert sind.

10. Verwendung der nach dem Verfahren der Ansprüche 1-9 hergestellten Antibiotika-Partikel zur antibiotischen Ausrüstung von Medizinprodukten und Arzneimitteln, die zur lokalen Freisetzung von einem Antibiotikum oder mehreren Antibiotika bestimmt sind.

11. Verwendung der nach dem Verfahren der Ansprüche 1-9 hergestellten Antibiotika-Partikel zur antibiotischen Ausrüstung von Polymethylmethacrylat-Knochenzement.
